(19) Europäisches Patentamt
European Patent Office
Office européen des brevets

(11) Numéro de publication : **0 110 747**
**B1**

(12) # FASCICULE DE BREVET EUROPÉEN

(45) Date de publication du fascicule du brevet : **28.05.86**

(51) Int. Cl.⁴ : **C 07 D211/14**, A 61 K 31/445

(21) Numéro de dépôt : **83402069.5**

(22) Date de dépôt : **25.10.83**

(54) **Dérivés de (2,4,6-triméthoxyphényl-(3-pipéridinopropyl)-cétone, utilisation en thérapeutique et procédé de préparation.**

(30) Priorité : **26.10.82 FR 8217938**

(43) Date de publication de la demande : **13.06.84 Bulletin 84/24**

(45) Mention de la délivrance du brevet : **28.05.86 Bulletin 86/22**

(84) Etats contractants désignés : **AT BE CH DE FR GB IT LI LU NL SE**

(56) Documents cités :
**GB-A- 1 115 992**
**GB-A- 1 218 583**
**US-A- 3 895 030**
**US-A- 4 031 101**

(73) Titulaire : **LABORATOIRE L. LAFON Société anonyme dite:**
**1 rue Georges Médéric**
**F-94701 Maisons-Alfort (FR)**

(72) Inventeur : **Lafon, Louis**
**5 rue de l'Alboni**
**F-75016 Paris (FR)**

(74) Mandataire : **Combe, André et al**
**CABINET BEAU DE LOMENIE 55 rue d'Amsterdam**
**F-75008 Paris (FR)**

**EP 0 110 747 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a trait à des dérivés de (2,4,6-triméthoxyphényl)-(3-pipéridinopropyl)-cétone en tant que produits industriels nouveaux. L'invention concerne également l'utilisation en thérapeutique et le procédé de synthèse de ces nouveaux dérivés.

On sait que l'on a déjà proposé dans le passé un certain nombre de composés du type phényl-aminoalkyl-cétone où le reste amino est notamment un groupe pipéridino. En particulier, on connaît :

— du brevet français n° 1 492 256, du brevet français (BSM) n° 5636M et de l'article de A. Boucherle et al., Chimie Thérapeutique, *3* (n° 4), 256-259 (1968), les (2,4,6-triméthoxyphényl)-(2-pipéridinoéthyl)-cétone, (2,4,6-triméthoxyphényl)-[2-(4-méthylpipéridino)-éthyl]-cétone et (2,4-diméthoxyphényl)-[2-(2-méthylpipéridino)-éthyl]-cétone qui présentent des effets essentiellement anti-inflammatoires, antalgiques et antipyrétiques, d'une part, et la (2,4,6-triméthoxyphényl)-[2-méthylpipéridino)-méthyl]-cétone, qui est un agent neuroleptique, d'autre part ;

— du brevet britannique n° 1 115 992, les (2,4-diméthoxyphényl)-(pipéridinométhyl)-cétone et (2,4,6-triméthoxyphényl)-[(4-méthylpipéridino)-méthyl]-cétone, qui présentent des effets essentiellement antispasmodiques et tranquillisants ; et

— du brevet américain n° 3 895 030, le chlorhydrate de (2,4,6-triméthoxyphényl)-(3-pipéridinopropyl)-cétone, qui présente des effets essentiellement antispasmodiques et qui est utile dans le traitement des coliques néphrétiques, et le chlorhydrate de (2,4,6-triméthoxyphényl)-(3-pyrrolidinopropyl)-cétone (n° de code LL 1656), qui est un agent vasodilatateur périphérique de référence, qui a fait l'objet d'une publication par Debray et al., Thérapie, *30*, 259-266 (1975) et qui est commercialisé en pharmacie sous le nom de « Fonzylane » (Dénomination Commune Internationale : Chlorhydrate de Buflomedil).

On sait enfin, notamment du brevet britannique n° 2 004 883 et de la demande de brevet européen n° 82 400 577.1, qu'il n'y a pas de relation structure-activité à l'intérieur de la famille des (alkoxy- et hydroxy-phényl)-aminoalkyl-cétones, les effets pharmacologiques étant modifiés ou disparaissant en fonction de la nature des substituants du groupe phényle, de la nature du groupe amino et, enfin, de la nature du groupe alkyle présent dans le groupe CO et le groupe amino. On sait en particulier de la demande de brevet européen précitée que le chlorhydrate de (2,6-diméthoxy-4-hydroxyphényl)-(3-pipéridinopropyl)-cétone (n° de code : CRL 40 746) est un agent vasodilatateur alors que son isomère le chlorhydrate de (2,4-diméthoxy-6-hydroxyphényl)-(3-pipéridinopropyl)-cétone (n° de code : CRL 40 747) est dépourvu d'intérêt pharmacologique en tant que substance vasodilatatrice par voie intraveineuse.

On vient de trouver de façon surprenante que les nouveaux dérivés de formule I ci-après et leurs sels d'addition (i) présentent des propriétés vasodilatatrices, hypotensives et bradycardisantes bénéfiques en thérapeutique, notamment dans le traitement des troubles de la circulation, (ii) se distinguent de leurs homologues antérieurement connus du type (2,4,6-triméthoxyphényl)-(pipéridinoalkyl)-cétone par leurs propriétés vasodilatatrices, les produits selon l'invention ayant un effet vasodilatateur fémoral par voie intraduodénale chez le chien anesthésié alors que lesdits homologues étudiés dans les mêmes conditions n'ont pas d'effet vasodilatateur fémoral.

Les nouveaux dérivés de (2,4,6-triméthoxyphényl)-(3-pipéridinopropyl)-cétone selon l'invention sont caractérisés en ce qu'ils sont choisis parmi l'ensemble constitué par

(i) les (2,4,6-triméthoxyphényl)-[3-(alkylpipéridino)-propyl]-cétones répondant à la formule générale I

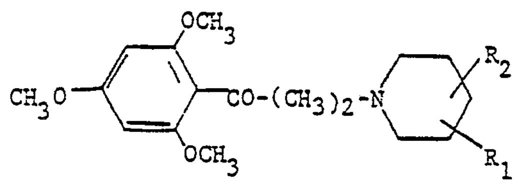

(I)

dans laquelle

$R_1$ représente un groupe $CH_3$ ou $C_2H_5$ uniquement en position 2 ou 3 du groupe pipéridino, et

$R_2$ représente H, $CH_3$ ou $C_2H_5$ ; et

(ii) leurs sels d'addition.

Les produits préférés selon l'invention sont le composé où $R_1$ = 3—$CH_3$ et $R_2$ = H, le composé où $R_1$ = 3—$CH_3$ et $R_2$ = 5—$CH_3$, et leurs sels.

Par sels d'addition, on entend ici, d'une part, les sels d'addition d'acide obtenus par réaction d'une base libre de formule I avec les acides minéraux et organiques et, d'autre part, les sels d'ammonium. Parmi les acides utilisables pour salifier les bases de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. Les sels d'addition d'acide sont les sels préférés et, parmi ces derniers, les chlorhydrates sont très intéressants sur le plan thérapeutique.

Les composés selon l'invention sont utiles dans le traitement des maladies cardiovasculaires, notamment en tant qu'agents vasodilatateurs, hypotenseurs et bradycardisants. Ce sont de bons médicaments des maladies liées aux troubles circulatoires et notamment du syndrome de Raynaud.

Selon l'invention, on préconise une composition thérapeutique qui renferme, en association avec un excipient physiologiquement acceptable, au moins une (2,4,6-triméthoxyphényl)-[3-(alkylpipéridino)-propyl]-cétone de formule I ou l'un de ses sels d'addition non toxiques, en tant qu'ingrédient actif. Dans une telle compositon, l'ingrédient actif est utilisé à une dose pharmaceutiquement efficace.

Les composés de formule I peuvent être préparés selon une méthode connue en soi par application de mécanismes réactionnels classiques. Le procédé que l'on préconise consiste

a) à faire réagir une pipéridine de formule II

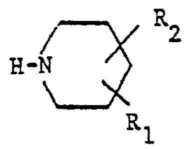

(II)

(où $R_1$ et $R_2$ sont définis comme ci-dessus) avec le 4-chloro-butyronitrile de formule III

$$Cl—(CH_2)_3—CN \qquad (III)$$

pendant au moins 2 h à reflux (de préférence à raison de 2 moles de II pour 1 mole de III), en présence d'un hydrocarbure aromatique tel que le benzène, le toluène et leurs mélanges, pour obtenir le 4-(akylpipéridino)-butyronitrile correspondant de formule IV

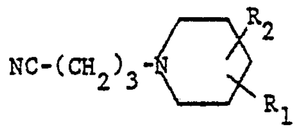

(IV)

(où $R_1$ et $R_2$ sont définis comme ci-dessus) ; et

b) à faire réagir le composé 4-(alkylpipéridino)-butyronitrile ainsi obtenu avec le 1,3,5-triméthoxybenzène en présence d'un courant gazeux de HCl à une température comprise entre — 5 °C et + 5 °C pendant au moins 2 h dans un solvant anhydre (notamment choisi parmi l'ensemble constitué par le benzène, le toluène, le chlorobenzène et leurs mélanges), puis à soumettre le dérivé cétimine ainsi formé à une réaction d'hydrolyse pendant au moins 0,5 h à la température de reflux du milieu réactionnel.

De façon avantageuse, la réaction du stade b) entre le 4-(alkylpipéridino)-butyronitrile et le 1,3,5-triméthoxybenzène sera mise en œuvre dans des conditions stœchiométriques au sein du chlorobenzène, le dérivé cétimine formé étant ensuite hydrolysé sans avoir été isolé du milieu réactionnel.

On a consigné dans les tableaux I, II et III ci-après un certain nombre

— de produits selon l'invention (exemple 1 à exemple 5) nullement limitatifs mais donnés à titre d'illustration,

— de produits de comparaison (CP-1 à CP-12) et

— de produits intermédiaires (A-1 à A-5) intervenant dans la synthèse des produits selon l'invention.

Tableau I

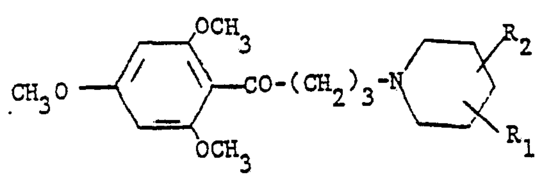

| Produit | n° de code | $R_1$ | $R_2$ | Solvant de recristallisation | Point de fusion |
|---|---|---|---|---|---|
| Exemple 1 (a) | CRL 41 008 | $2-CH_3$ | H | (b) | 163°C |
| Exemple 2 (a) | CRL 41 034 | $3-CH_3$ | H | (c) | 110°C |
| Exemple 3 (a) | CRL 41 035 | $2-C_2H_5$ | H | (d) | 130°C |
| Exemple 4 (a) | CRL 41 043 | $3-CH_3$ | $5-CH_3$ | (d) | 167°C |
| Exemple 5 (a) | CRL 41 044 | $2-CH_3$ | $6-CH_3$ | (d) | 148°C |
| CP-1 (a)(f) | - | H | H | (e) | 210-215°C |
| CP-2 (a) (g) | CRL 41 007 | $4-CH_3$ | H | (b) | 160°C |

Notes
(a) : chlorhydrate
(b) : isopropanol
(c) : isopropanol-acétate d'éthyle (1 : 5) v/v
(d) : isopropanol-acétate d'éthyle (1 : 3) v/v
(e) : éthanol-eau (50 : 1) v/v
(f) : décrit à l'exemple 14 du brevet américain n° 3 895 030
(g) : suggéré par le brevet américain n° 3 895 030

Tableau II

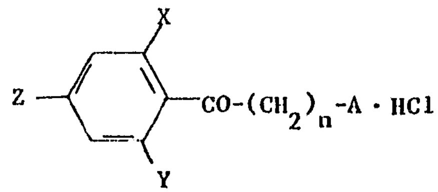

| Produit | N° de code | X | Y | Z | n | A |
|---|---|---|---|---|---|---|
| CP-3 (a) | - | $OCH_3$ | $OCH_3$ | $OCH_3$ | 2 | pipéridino |
| CP-4 (a) | - | $OCH_3$ | $OCH_3$ | $OCH_3$ | 2 | 4-méthylpipéridino |
| CP-5 (b) | - | $OCH_3$ | H | $OCH_3$ | 1 | pipéridino |
| CP-6 (b) | - | $OCH_3$ | $OCH_3$ | $OCH_3$ | 1 | 4-méthylpipéridino |
| CP-7 (c) | - | OH | OH | OH | 3 | pipéridino |
| CP-8 (d) | CRL 40 747 | $OCH_3$ | $OCH_3$ | OH | 3 | pipéridino |
| CP-9 (e) | - | $OCH_3$ | $OCH_3$ | $OCH_3$ | 1 | 2-méthylpipéridino |
| CP-10(e) | - | $OCH_3$ | H | $OCH_3$ | 2 | 2-méthylpipéridino |
| CP-11(d) | CRL 40 746 | $OCH_3$ | OH | $OCH_3$ | 3 | pipéridino |
| CP-12(c) | LL 1656 | $OCH_3$ | $OCH_3$ | $OCH_3$ | 3 | pyrrolidino |

Notes
(a) décrit dans brevets français n° 1 492 256 et n° 5 636M
(b) décrit dans brevet britannique n° 1 115 992
(c) décrit dans brevet américain n° 3 895 030
(d) décrit dans demande européenne n° 82 400 5771.
(e) décrit par Boucherle et al., Chimie Thérapeutique 3 (n° 4), pages 256-259 (1968)

Tableau III

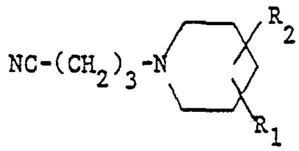

| Produit | Intermédiaire de synthèse pour | $R_1$ | $R_2$ | $Eb_{5-6\ mmHg}$ |
|---|---|---|---|---|
| A-1 | exemple 1 | $2-CH_3$ | H | 102°C |
| A-2 | exemple 2 | $3-CH_3$ | H | 100°C |
| A-3 | exemple 3 | $2-C_2H_5$ | H | 115-116°C |
| A-4 | exemple 4 | $3-CH_3$ | $5-CH_3$ | 109-110°C |
| A-5 | exemple 5 | $2-CH_3$ | $6-CH_3$ | 114-116°C |

Note
5-6 mmHg correspondent approximativement à 666-800 pascals

D'autres avantages et caractéristiques de l'invention seront mieux compris à la lecture qui va suivre d'exemples de préparation nullement limitatifs, d'une part, et du résumé des essais qui ont été entrepris notamment sur le plan pharmacologique, d'autre part.

Préparation I

Obtention du chlorhydrate de (2,4,6-triméthoxyphényl)-[3-(3-méthylpipéridino)-propyl]-cétone

(Exemple 2 ; n° de code : CRL 41 034)

a) 4-(3-méthylpipéridino)-butyronitrile

Au sein d'une solution au reflux de 58,6 ml (0,50 mole) de 3-méthylpipéridine dans 65 ml de benzène, on coule en 25 minutes 27,2 g (0,25 mole) de 4-chlorobutyronitrile. On poursuit le reflux 2 h, on élimine le précipité par filtration et on amène le filtrat à siccité sous pression réduite. Le résidu d'évaporation est purifié par distillation sous pression réduite pour donner 29,2 g (rendement = 70,3 %) de 4-(3-méthylpipéridino)-butyronitrile qui se présente sous la forme d'une huile incolore. $Eb_{5-6\ mmHg}$ = 100 °C.

b) CRL 41 034

Au sein d'une solution maintenue à environ 0 °C et comprenant 20,2 g (0,12 mole) de 1,3,5-triméthoxybenzène et 20 g (0,12 mole) de 4-(3-méthylpipéridino)-butyronitrile dans 100 ml de chlorobenzène anhydre, ont fait passer pendant 2,5 h à une température d'environ 0 °C. On extrait le milieu réactionnel par de l'eau, porte au reflux pendant 1 h la phase aqueuse. On lave avec de l'éther diéthylique, alcanilise avec $NH_4OH$ et extrait le précipité formé au moyen d'éther diéthylique. La phase organique résultante qui renferme la base libre, à savoir la (2,4,6-triméthoxyphényl)-[3-(3-méthylpipéridino)-propyl]-cétone, est traitée avec de l'éthanol chlorhydrique, le précipité obtenu est purifié par recristallisation du mélange isopropanol-acétate d'éthyle (1 : 5) v/v pour donner 27 g [rendement du stade b) : 60,5 %, rendement global : 42,5 %] de CRL 41 034 qui se présente sous la forme d'une poudre blanche soluble dans l'eau. $F_{inst}$(Kofler) = 110 °C.

Préparation II

Obtention du chlorhydrate de (2,4,6-triméthoxyphényl)-[3-(3,5-diméthylpipéridino)-propyl]-cétone

(Exemple 4 ; n° de code : CRL 41 043)

a) 4-(3,5-diméthylpipéridino)-butyronitrile

Au sein d'une solution au reflux de 50 g (0,424 mole) de 3,5-diméthylpipéridine à 96 % dans 60 ml de

benzène, on coule en 25 minutes 22 g (0,212 mole) de 4-chlorobutyronitrile et on poursuit le reflux pendant 2 h. On élimine le précipité par filtration et on amène à siccité le filtrat par évaporation sous vide. Le résidu d'évaporation est purifié par distillation sous pression réduite pour donner 26 g (rendement = 68,13 %) de 4-(3,5-diméthylpipéridino)-butyronitrile se présentant sous la forme d'une huile incolore. $Eb_{5-6\ mmHg}$ = 109-110 °C.

b) CRL 41 043

Au sein d'une solution maintenue vers 0 °C de 20,2 g (0,12 mole) de 1,3,5-triméthoxybenzène et de 21,6 g (0,12 mole) de 4-(3,5-diméthylpipéridino)-butyronitrile dans 100 ml de chlorobenzène anhydre, on fait passer pendant 2 h un courant d'acide chlorhydrique gazeux sec et on abandonne en glacière pendant 2 h. On extrait le milieu réactionnel par de l'eau, puis on porte la phase aqueuse au reflux pendant 1,5 h. On alcalinise par de la soude et on extrait le précipité par de l'éther diéthylique pour obtenir 34,1 g de (2,4,6-triméthoxyphényl-[3-(3,5-diméthylpipéridino)-propyl]-cétone qui se présente sous la forme d'une huile légèrement orangée. La base libre est traitée dans de l'éther diéthylique par de l'éthanol chlordhydrique. On obtient après purification du précipité au moyen de deux recristallisations successives du mélange isopropanol-acétate d'éthyle (1 : 3) v/v 16 g [rendement du stade b) : 34,6 % ; rendement global : 23,6 %] de CRL 41 043 qui se présente sous la forme d'une poudre blanche soluble dans l'eau. $F_{inst}$ (Kofler) = 167 °C.

Préparation III

Obtention du chlorhydrate de (2,4,6-triméthoxyphényl)-[3-(2,6-diméthylpipéridino)-propyl]-cétone

(Exemple 5 ; n° de code : CRL 41 044)

a) 4-(2,6-diméthylpipéridino)-butyronitrile

On chauffe au reflux pendant 4 h une solution de 50 g (0,438 mole) de 2,6-diméthylpipéridine et de 22,6 g (0,219 mole) de 4-chlorobutyronitrile dans 50 ml de benzène. On élimine le précipité par filtration et on amène à siccité le filtrat. Le résidu d'évaporation ainsi obtenu est purifié par distillation sous pression réduite pour donner 11 g (rendement = 27,9 %) de 4-(2,6-diméthylpipéridino)-butyronitrile se présentant sous la forme d'une huile incolore. $Eb_{5-6\ mmHg}$ = 114-116 °C.

b) CRL 41 044

Au sein d'une solution maintenue vers 0 °C de 18,2 g (0,108 mole) de 1,3,5-triméthoxybenzène et de 19,5 g (0,108 mole) de 4-(2,6-diméthylpipéridino)-butyronitrile dans 100 ml de chlorobenzène anhydre, on fait passer pendant 2,5 h un courant d'acide chlorhydrique gazeux sec et on abandonne une nuit en glacière. On extrait le milieu réactionnel par de l'eau et on porte la phase aqueuse au reflux pendant 1,5 h. On alcanalise par de la soude et on extrait le précipité par de l'éther diéthylique pour obtenir 34,2 g de (2,4,6-triméthoxyphényl)-[3-(2,6-diméthylpipéridino)-propyl]-cétone sous la forme d'une huile orangée. La base libre huileuse est traitée dans de l'éther diéthylique par de l'éthanol chlorhydrique. On obtient après purification du précipité par recristallisation du mélange isoprapanol-acétate d'éthyle (1 : 3) v/v 9,5 g [rendement du stade b) : 22,8 % ; rendement global : 6,4 %] de CRL 41 044 qui se présente sous la forme d'une poudre blanche ayant une solubilité dans l'eau de l'ordre de 100 g/l. $F_{inst}$ (Kofler) = 148 °C.

On a résumé ci-après les essais qui ont été entrepris avec les produits selon l'invention.

A — Essais comparatifs

On a comparé les produits selon l'invention avec leurs analogues structuraux (CP-1 à CP-11) et un agent vasodilatateur périphérique de référence qui est le LL 1656 (CP-12) précité.

Les propriétés vasodilatatrices périphériques ont été étudiées chez le chien mâle anesthésié au nembutal (6 animaux par dose et par produit). Les produits à comparer ont été administrés en solution dans du sérum physiologique sous un volume de 6 ml/animal par voie intraveineuse (perfusion de 1 ml/min). Par rapport aux témoins (les mêmes animaux ne recevant que le sérum physiologique), on mesure trois paramètres : la pression artérielle moyenne (exprimée en mmHg ; 1 mmHg correspond à 1,333 224 × $10^2$ Pa), la fréquence cardiaque (exprimée en battements/minute) et le débit artériel fémoral (exprimé en ml/min). Les variations de ces paramètres exprimées en pourcentages, par rapport aux témoins, sont données dans le tableau IV ci-après.

Les résultats du tableau IV montrent l'intérêt des produits selon l'invention Ex 1 à Ex 5 par rapport à leurs homologues pipéridino CP-1 à CP-11, d'une part, et par rapport au produit du type pyrrolidino CP-12, d'autre part.

Tableau IV

Variation des paramètres après administration intraveineuse chez le chien anesthésié

| Produit | No de code | Dose (mg/kg) | Variations en % Pression artérielle | Débit artériel fémoral | Fréquence cardiaque |
|---|---|---|---|---|---|
| Ex.1 | CRL 41 008 | 1,5 | 0 | + 8 | - 10 |
| Ex.2 | CRL 41 034 | 1,5 | - 2 | + 5 | + 14 |
| Ex.3 | CRL 41 035 | 1,5 | + 5 | + 47 | + 2 |
| Ex.4 | CRL 41 043 | 1,5 | + 5 | + 53 | + 5 |
| Ex.5 | CRL 41 044 | 1,5 | + 3 | + 27 | - 1 |
| CP - 1 | - | 1,5 | + 1 | + 10 | - 2 |
| CP - 2 | CRL 41 007 | 1,5 | + 1 | + 8 | - 1 |
| CP - 3 | - | 1,5 | + 3 | + 8 | - 1 |
| CP - 4 | - | 1,5 | - 2 | + 3 | - 2 |
| CP - 5 | - | 1,5 | - 1 | - 5 | + 1 |
| CP - 6 | - | 1,5 | + 1 | + 7 | + 3 |
| CP - 7 | - | 1,5 | + 1 | + 10 | - 2 |
| CP - 8 | CRL 40 747 | 1,5 | + 2 | + 20 | + 5 |
| CP - 9 | - | 1,5 | - 1 | - 4 | + 1 |
| CP - 10 | - | 1,5 | - 2 | + 2 | - 1 |
| CP - 11 | CRL 40 746 | 1,5 | + 2 | + 34 | + 2 |
| CP - 12 | LL 1656 | 1,5 | 0 | + 33 | + 5 |
| Ex. 1 | CRL 41 008 | 2 | 0 | + 22 | - 5 |
| Ex. 2 | CRL 41 034 | 2 | - 6 | + 24 | + 19 |
| Ex. 3 | CRL 41 035 | 2,5 | + 6 | + 45 | 0 |
| Ex. 4 | CRL 41 043 | 2 | - 2 | + 87 | - 14 |
| Ex. 5 | CRL 41 044 | 2,5 | + 5 | + 21 | 0 |
| CP - 1 | - | 3 | + 1 | + 18 | + 3 |
| CP - 2 | CRL 41 007 | 3 | + 2 | + 10 | + 2 |
| CP - 3 | - | 3 | - 2 | + 8 | - 6 |
| CP - 4 | - | 3 | - 5 | + 2 | + 1 |
| CP - 5 | - | 3 | - 8 | - 8 | + 3 |
| CP - 6 | - | 3 | - 1 | + 5 | - 2 |
| CP - 7 | - | 3 | + 1 | + 18 | + 3 |
| CP - 8 | CRL 40 747 | 3 | - 12 | + 22 | - 2 |
| CP - 9 | - | 3 | - 1 | + 4 | - 1 |
| CP - 10 | - | 3 | - 3 | + 3 | + 2 |
| CP - 11 | CRL 40 746 | 3 | + 5 | + 35 | + 3 |
| CP - 12 | LL 1656 | 3 | - 2 | + 36 | + 6 |
| Ex. 1 | CRL 41 008 | 5 | - 4 | + 40 | - 5 |
| Ex. 2 | CRL 41 034 | 5 | - 25 | + 65 | - 2 |
| Ex. 3 | CRL 41 035 | 5 | - 2 | + 79 | - 8 |
| Ex. 4 | CRL 41 043 | 4 | - 15 | + 40 | - 26 |
| Ex. 5 | CRL 41 044 | 5 | + 1 | + 46 | - 7 |
| CP - 1 | - | 6 | + 2 | + 34 | + 3 |
| CP - 2 | CRL 41 007 | 6 | + 3 | + 18 | + 2 |
| CP - 3 | - | 6 | + 5 | + 3 | - 1 |
| CP - 4 | - | 6 | + 1 | + 2 | - 1 |
| CP - 5 | - | 6 | - 2 | - 3 | + 2 |
| CP - 6 | - | 6 | - 8 | + 2 | + 1 |
| CP - 7 | - | 6 | + 2 | + 34 | + 3 |
| CP - 8 | CRL 40 747 | 6 | - 18 | + 22 | - 15 |
| CP - 9 | - | 6 | - 3 | - 2 | + 2 |
| CP - 10 | - | 6 | + 3 | + 2 | - 2 |
| CP - 11 | CRL 40 746 | 6 | + 5 | + 41 | + 3 |
| CP - 12 | LL 1656 | 6 | - 2 | + 39 | 0 |

Les propriétés vasodilatatrices par voie intraduodénale ont été étudiées chez le chien anesthésié au nembutal. Selon le protocole opératoire donné ci-dessus, les produits à tester ont été administrés en solution dans du sérum physiologique sous un volume de 10 ml/animal par voie intraduodénale. On observe que, selon ce mode d'administration, une nette augmentation du débit artériel fémoral à partir de 2,5 mg/kg pour le produit de l'exemple 4 et à partir de 5 mg/kg pour les produits des exemples 1-3 et 5, alors qu'il faut des doses de 20 mg/kg pour les CRL 40 746 (CP-11) et LL 1656 (CP-12) pour avoir la même augmentation. On observe également, après administration intraduodénale, qu'un effet hypotenseur et un effet bradycardisant se manifestent à partir de la dose de 5 mg/kg pour le produit de l'exemple 4 et à partir de la dose de 10 mg/kg pour les produits des exemples 1-3 et 5, alors que la dose de 20 mg/kg est nécessaire pour obtenir les mêmes effets avec CP-11 et CP-12.

B — Essais relatifs au CRL 41 034 (exemple 2)

I — Action vasodilatatrice fémorale par voie intraveineuse

Deux chiens (poids moyen : 10,4 kg) anesthésiés au pentobarbital reçoivent le CRL 41 034 par voie intraveineuse en perfusion de 6 minutes, aux doses successives de 1, 2 et 4 mg/kg séparées les unes des autres par environ 1 heure. L'un reçoit une dose supplémentaire de 8 mg/kg. Pour comparaison, ces animaux reçoivent également une perfusion de 6 mg/kg I.V. de LL 1656.

On observe avec le CRL 41 034 une tachycardie dès la dose de 1 mg/kg. Une nette augmentation du débit fémoral apparaît à 4 mg/kg ; elle dure plus de 15 minutes et disparaît à 60 minutes ; elle est accompagnée d'une hypotension. L'effet de 2 mg/kg de CRL 41 034 sur le débit fémoral est en moyenne comparable à l'effet de 6 mg/kg I.V. de LL 1656.

Chez un chien, le LL 1656 est perfusé à 6 mg/kg en début d'essai et cette dose a le même effet, sur le débit fémoral, que 2 mg/kg de CRL 41 034 (+ 60 % à 6 minutes, + 40 % à 15 minutes, et disparition de l'effet à 30 minutes).

Chez l'autre chien, le LL 1656 est perfusé à 6 mg/kg en fin d'essai après que le chien a reçu au total 15 mg/kg I.V. de CRL 41 034 et à un moment où le débit fémoral est encore augmenté par le CRL 41 034. On constate que le CRL 41 034 augmente et prolonge l'effet du LL 1656 sur le débit fémoral puisque, à 30 minutes, l'augmentation du débit fémoral (par rapport à sa valeur avant tout traitement) est encore de 150 %.

II — Action vasodilatatrice par voie intraduodénale

Trois chiens (poids moyen : 13,5 kg) anesthésiés au nembutal reçoivent le CRL 41 034 par voie intraduodénale, aux doses successives de 0,1, 0,5, 1, 2, 5 et 10 mg/kg.

On mesure la pression artérielle, la fréquence cardiaque, le débit artériel fémoral, le débit artériel vertébral, la température rectale, et on note la coloration de la peau et la coloration de la bile recueillie par cathétérisation du cholédoque après ligature du cystique.

On observe une augmentation du débit fémoral importante à partir de la dose de 5 mg/kg. Un effet hypotenseur et une bradycardie apparaissent à 10 mg/kg. On ne note aucune augmentation du débit vertébral. Les températures rectale et cutanée ne varient pas ; on ne constate aucune modification de la peau et de la bile.

C — Essais relatifs au CRL 41 035 (exemple 3)

Deux chiens (poids moyen : 14,5 kg) anesthésiés au nembutal reçoivent le CRL 41 035 par voie intraduodénale, aux doses successives de 0,5 mg/kg, 1 mg/kg, 2,5 mg/kg, 5 mg/kg et 10 mg/kg ; un chien reçoit une dose supplémentaire de 10 mg/kg I.V.

On mesure la pression artérielle, la fréquence cardiaque, le débit artériel fémoral, le débit artériel vertébral, la température rectale. On observe la coloration de la peau et la coloration de la bile recueillie par cathétérisation du cholédoque après ligature du cystique.

Le CRL 41 035 étudié sur ces deux chiens anesthésiés se montre hypotenseur à la dose de 10 mg/kg I.D. par la baisse de la pression artérielle systolique et bradycardisante. On note une augmentation du débit fémoral à 5 mg/kg qui reste présente à 10 mg/kg malgré la forte hypotension ; de ce fait, la résistance fémorale diminue. Aucune vasodilatation vertébrale n'est observée. La dose supplémentaire de 10 mg/kg injectée à un chien confirme l'hypotension — 41 % (139 à 82 mmHg) à 30 minutes, la bradycardie — 35 % (155 à 100 battements/minute) et l'augmentation modérée mais encore présente du débit fémoral + 12 % (43 à 48 ml/min).

Les températures rectale et cutanée ne varient pas. La coloration de la peau et de la bile n'est pas modifiée.

Les effets de l'isoprénaline testés après la dose cumulée de 19 mg/kg sont légèrement diminués sur la fréquence cardiaque et augmentés sur la pression artérielle diastolique. A 3 mcg/kg d'isoprénaline, la pression artérielle diastolique passe à 32 mmHg au lieu de 56 mmHg et la fréquence cardiaque passe à 210 battements/minute au lieu de 245 battements/minute en valeur témoin.

L'hypertension à la noradrénaline est fortement diminuée sur la pression artérielle systolique. A 2 mcg/kg de noradrénaline, la pression artérielle systolique passe à 196 mmHg au lieu de 256 mmHg.

D — Essais relatifs au CRL 41 043 (exemple 4)

I — Action vasodilatatrice fémorale par voie intraveineuse

Quatre chiens (poids moyen 11,7 kg) anesthésiés au nembutal reçoivent le CRL 41 043 par voie intraveineuse, en perfusion de 6 min, aux doses successives de 1 et 2 mg/kg ; trois de ces chiens reçoivent ensuite 6 mg/kg de LL 1656, puis 4 mg/kg de CRL 41 043.

Le CRL 41 043, à 1 mg/kg, augmente le débit fémoral pendant la durée de la perfusion sans modifier nettement les autres paramètres.

La dose de 2 mg/kg a un effet plus intense et plus durable sur le débit fémoral qui augmente pendant 15 min. La dose supplémentaire de 4 mg/kg augmente moins le débit fémoral et entraîne hypotension et bradycardie. Le LL 1656, 6 mg/kg I.V., a le même effet sur le débit fémoral que 2 mg/kg I.V. de CRL 41 043.

II — Action vasodilatatrice fémorale par voie intraduodénale

Trois chiens (poids moyen 18,3 kg) anesthésiés au nembutal reçoivent le CRL 41 043 par voie intraduodénale, aux doses successives de 0,1 mg/kg, 0,5 mg/kg, 1 mg/kg, 2,5 mg/kg et 5 mg/kg.

On observe que le CRL 41 043 augmente le débit fémoral à partir de la dose de 2,5 mg/kg. Une hypotension et une bradycardie apparaissent à partir de la dose de 5 mg/kg, l'augmentation du débit fémoral est restaurée lorsque l'hypotension disparaît. Aucune augmentation du débit vertébral n'est observée. Les températures rectale et cutanée diminuent modérément. A la dose de 5 mg/kg on constate que l'augmentation du débit fémoral est maximale 30 min après administration, l'effet vasodilatateur fémoral disparaissant 1 h après administration.

E — Essais cliniques

Chez l'homme, le CRL 41 034 (exemple 2) sous forme de comprimés ou gélules renfermant chacun 0,75 g de principe actif, s'est avéré, à raison de 2 à 3 comprimés ou gélules par jour, un bon médicament des troubles circulatoires et notamment du syndrome de Raynaud.

Chez l'homme, le CRL 41 043 (exemple 4) sous forme de comprimés renfermant chacun 0,50 g de principe actif s'est avéré, à raison de 2 à 3 comprimés par jour, un bon médicament vasodilatateur périphérique.

**Revendications** (pour les Etats contractants : BE, CH, DE, FR, GB, IT, LI, LU, NL, SE)

1. Dérivé de (2,4,6-triméthoxyphényl)-(3-pipéridinopropyl)-cétone, caractérisé en ce qu'il est choisi parmi l'ensemble constitué par

(i) les (2,4,6-triméthoxyphényl)-[3-(alkylpipéridino)-propyl]-cétones répondant à la formule générale I

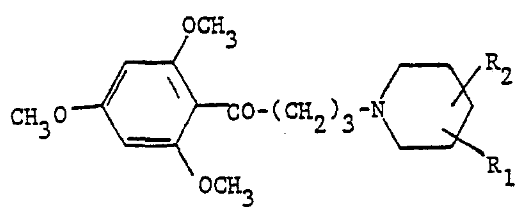

(I)

dans laquelle

$R^1$ représente un groupe $CH_3$ ou $C_2H_5$ en position 2 ou 3 du groupe pipéridino et

$R_2$ représente H, $CH_3$ ou $C_2H_5$ ; et

(ii) leurs sels d'addition.

2. (2,4,6-Triméthoxyphényl)-[3-(2-méthylpipéridino)-propyl]-cétone et ses sels d'addition.
3. (2,4,6-Triméthoxyphényl)-[3-(3-méthylpipéridino)-propyl]-cétone et ses sels d'addition.
4. (2,4,6-Triméthoxyphényl)-[3-(2-éthylpipéridino)-propyl]-cétone et ses sels d'addition.
5. (2,4,6-Triméthoxyphényl)-[3-(3,5-diméthylpipéridino)-propyl]-cétone et ses sels d'addition.
6. (2,4,6-Triméthoxyphényl)-[3-(2,6-diméthylpipéridino)-propyl]-cétone et ses sels d'addition.
7. Composition thérapeutique, caractérisée en ce qu'elle renferme, en association avec un excipient physiologiquement acceptable, au moins un dérivé de (2,4,6-triméthoxyphényl)-[3-(alkylpipéridino)-propyl]-cétone ou l'un de ses sels d'addition non toxiques selon l'une quelconque des revendications 1 à 6.

8. Procédé de préparation d'un composé de formule I selon la revendication 1, dans lequel on forme un 4-(alkylpipéridino)-butyronitrile que l'on condense avec le 1,3,5-triméthoxybenzène et hydrolyse le dérivé cétimine résultant, ledit procédé étant caractérisé en ce que :

1°) on fait réagir une pipéridine substituée de formule II

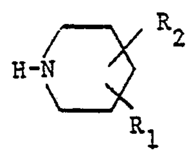

(II)

(où $R_1$ et $R_2$ sont définis comme ci-dessus) avec le 4-chlorobutyronitrile de formule III

$$Cl—(CH_2)_3—CN \quad (III)$$

pendant au moins 2 h à reflux dans un solvant choisi parmi les hydrocarbures aromatiques tels que, notamment le benzène, le toluène et leurs mélanges, à raison de 2 moles de II pour 1 mole de III, pour obtenir le 4-(alkylpipéridino)-butyronitrile correspondant de formule IV

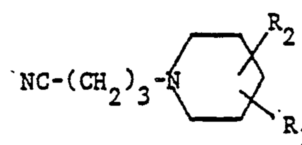

(IV)

(où $R_1$ et $R_2$ sont définis comme ci-dessus) ; et

2°) on fait réagir ledit 4-(alkylpipéridino)-butyronitrile avec le 1,3,5-triméthoxybenzène en présence d'un courant gazeux de HCl, à une température comprise entre — 5 et + 5 °C, dans un solvant anhydre choisi notamment parmi l'ensemble constitué par le benzène, le toluène, le chlorobenzène et leurs mélanges, à raison de 1 mole de IV pour 1 mole de 1,3,5-triméthoxybenzène, puis soumet le dérivé cétimine ainsi formé à une réaction d'hydrolyse pendant au moins 0,5 h à la température de reflux du milieu réactionnel.

**Revendications** (pour l'Etat contractant AT)

1. Utilisation d'un dérivé de (2,4,6-triméthoxyphényl)-(3-pipéridino-propyl)-cétone, choisi parmi l'ensemble constitué par

(i) les (2,4,6-triméthoxyphényl)-[3-(alkylpipéridino)-propyl]-cétones répondant à la formule générale I

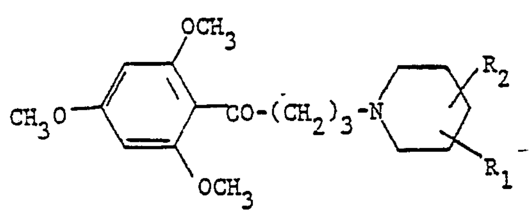

(I)

dans laquelle

$R_1$ représente un groupe $CH_3$ ou $C_2H_5$ en position 2 ou 3 du groupe pipéridino et

$R_2$ représente H, $CH_3$ ou $C_2H_5$ ; et

(ii) leurs sels d'addition non toxiques pour la fabrication d'un médicament.

2. Utilisation selon la revendication 1, caractérisée en ce que le dérivé est le (2,4,6-triméthoxyphényl)-[3-(2-méthylpipéridino)-propyl]-cétone et ses sels d'addition.

3. Utilisation selon la revendication 1, caractérisée en ce que le dérivé est le (2,4,6-triméthoxyphényl)-[3(3-méthylpipéridino)-propyl]-cétone, et ses sels d'addition.

4. Utilisation selon la revendication 1, caractérisée en ce que le dérivé est le (2,4,6-triméthoxyphényl)-[3-(2-éthylpipéridino)-propyl]-cétone et ses sels d'addition.

5. Utilisation selon la revendication 1, caractérisée en ce que le dérivé est le (2,4,6-triméthoxyphényl)-[3-(3,5-diméthylpipéridino)-propyl]-cétone et ses sels d'addition.

6. Utilisation selon la revendication 1, caractérisée en ce que le dérivé est le (2,4,6-triméthoxyphényl)-[3-(2,6-diméthylpipéridino)-propyl]-cétone et ses sels d'addition.

7. Utilisation selon l'une des revendications 1 à 6, caractérisée en ce que au moins un dérivé de (2,4,6-triméthoxyphényl)-[3-alkyl-pipéridino)-propyl]-cétone ou l'un de ses sels d'addition non toxiques est en association avec un excipient physiologiquement acceptable.

8. Procédé de préparation d'un composé de formule I selon la revendication 1, dans lequel on forme un 4-(alkylpipéridino)-butyronitrile que l'on condense avec le 1,3,5-triméthoxybenzène et hydrolyse le dérivé cétimine résultant, ledit procédé étant caractérisé en ce que :

1°) on fait réagir une pipéridine substituée de formule II

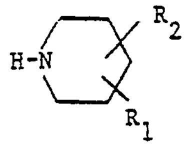

(II)

(où $R_1$ et $R_2$ sont définis comme ci-dessus) avec le 4-chlorobutyronitrile de formule III

$$Cl—(CH_2)_3—CN \quad (III)$$

pendant au moins 2 h à reflux dans un solvant choisi parmi les hydrocarbures aromatiques tels que, notamment le benzène, le toluène et leurs mélanges, à raison de 2 moles de II pour 1 mole de III, pour obtenir le 4-(alkylpipéridino)-butyronitrile correspondant de formule IV

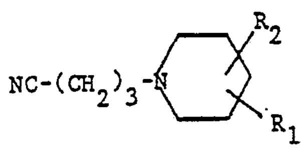

(IV)

(où $R_1$ et $R_2$ sont définis comme ci-dessus) ; et

2°) on fait réagir ledit 4-(alkylpipéridino)-butyronitrile avec le 1,3,5-triméthoxybenzène en présence d'un courant gazeux de HCl, à une température comprise entre — 5 et + 5 °C, dans un solvant anhydre choisi notamment parmi l'ensemble constitué par le benzène, le toluène, le chlorobenzène et leurs mélanges, à raison de 1 mole de IV pour 1 mole de 1,3,5-triméthoxybenzène, puis soumet le dérivé cétimine ainsi formé à une réaction d'hydrolyse pendant au moins 0,5 h à la température de reflux du milieu réactionnel.

**Claims** (for the Contracting States : BE, CH, DE, FR, GB, IT, LI, LU, NL, SE)

1. (2,4,6-trimethoxyphenyl)-(3-piperidinopropyl)-ketone derivate, characterized in that it is selected among the group constituted by

(i) the (2,4,6-trimethoxyphenyl)-[3-(alkylpiperidino)-propyl]-ketones responding to the general formula I

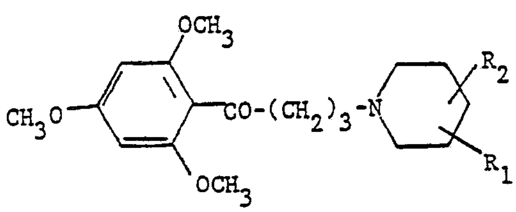

(I)

in which

$R_1$ is a $CH_3$ or $C_2H_5$ group in position 2 or 3 of the piperidino group, and

$R_2$ is H, $CH_3$ or $C_2H_5$ ; and

(ii) the addition salts thereof.

2. (2,4,6-Trimethoxyphenyl)-[3-(2-methylpiperidino)-propyl]-ketone and the addition salts thereof.

3. (2,4,6-Trimethoxyphenyl)-[3-(3-methylpiperidino)-propyl]-ketone and the addition salts thereof.

4. (2,4,6-Trimethoxyphenyl)-[3-(2-ethylpiperidino)-propyl]-ketone and the addition salts thereof.

5. (2,4,6-Trimethoxyphenyl)-[3-(3,5-dimethylpiperidino)-propyl]-ketone and the addition salts thereof.

6. (2,4,6-Trimethoxyphenyl)-[3-(2,6-dimethylpiperidino)-propyl]-ketone and the addition salts thereof.

7. Therapeutical composition, characterized in that it contains, in association with a physiologically acceptable excipient, at least one derivative of (2,4,6-trimethoxyphenyl)-[3-(alkylpiperidino)-propyl]-ketone or one the non toxic addition salts thereof according to any one of claims 1 to 6.

8. Process for preparing a compound of formula I according to claim 1, in which a 4-(alkylpiperidino)-butyronitrile is formed and condensed with the 1,3,5-trimethoxybenzene and the resulting ketimine derivative is hydrolyzed, said process being characterized in that :

1°) a substituted piperidine of formula II

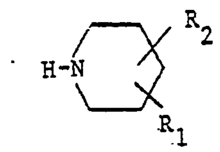

(II)

(wherein $R_1$ and $R_2$ are as defined above) is reacted with the 4-chlorobutyronitrile of formula III

$$Cl—(CH_2)_3—CN \quad (III)$$

for at least two hours at reflux in a solvent selected from the aromatic hydrocarbons such as in particular benzene, toluene and the mixtures thereof, at the rate of two moles of II for one mole of III, to obtain the corresponding 4-(alkylpiperidino)-butyronitrile of formula IV

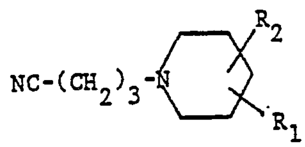

(IV)

(wherein $R_1$ and $R_2$ are defined as above) ; and

2°) said 4-(alkylpiperidino)-butyronitrile is reacted with the 1,3,5,-trimethoxybenzene in the presence of a gaseous stream of HCl, at a temperature of between — 5 and + 5 °C, in an anhydrous solvent selected in particular from the group constituted by benzene, toluene, chlorobenzene and the mixtures thereof, at the rate of one mole of IV for one mole of 1,3,5-trimethoxybenzene, then the ketimine derivative thus obtained is subjected to a reaction of hydrolysis for at least 0.5 hour at the reflux temperature of the reaction medium.

**Claims** (for the Contracting State AT)

1. Use of a derivative of (2,4,6-trimethoxyphenyl)-(3-piperidino-propyl)-ketone, selected from the group constituted by

(i) the (2,4,6-trimethoxyphenyl)-[3-(alkylpiperidino)-propyl]-ketones responding to the general formula I

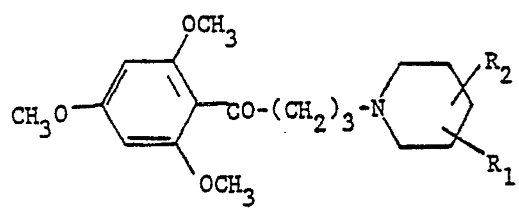

(I)

in which

$R_1$ is a $CH_3$ or $C_2H_5$ group in position 2 or 3 of the piperidino group, and

$R_2$ is H, $CH_3$ or $C_2H_5$ ; and

(ii) the non toxic addition salts thereof for the preparation of a drug.

2. Use according to claim 1, characterized in that the derivative is the (2,4,6-trimethoxyphenyl)-[3-(2-methylpiperidino)-propyl]-ketone and the addition salts thereof.

3. Use according to claim 1, characterized in that the derivative is the (2,4,6-trimethoxyphenyl)-[3-(3-methylpiperidino)-propyl]-ketone, and the addition salts thereof.

4. Use according to claim 1, characterized in that the derivative is the (2,4,6-trimethoxyphenyl)-[3-(2-ethylpiperidino)-propyl]-ketone and the addition salts thereof.

5. Use according to claim 1, characterized in that the derivative is the (2,4,6-trimethoxyphenyl)-[3-(3,5-dimethylpiperidino)-propyl]-ketone and the addition salts thereof.

6. Use according to claim 1, characterized in that the derivative is the (2,4,6-trimethoxyphenyl)-[3-(2,6-dimethylpiperidino)-propyl]-ketone and the addition salts thereof.

7. Use according to any one of claims 1 to 6, characterized in that at least one derivative of (2,4,6-trimethoxyphenyl)-[3-(alkylpiperidino)-propyl]-ketone or one of the non toxic addition salts thereof is in association with a physiologically acceptable excipient.

8. Process for preparing a compound of formula I according to claim 1, in which a 4-(alkylpiperidino)-butyronitrile is formed and condensed with the 1,3,5-trimethoxybenzene and the resulting ketimine derivative is hydrolyzed, said process being characterized in that :

1°) a substituted piperidine of formula II

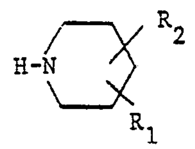

(II)

(wherein $R_1$ and $R_2$ are defined as above) is reacted with the 4-chlorobutyronitrile of formula III

$$Cl—(CH_2)_3—CN \quad (III)$$

for at least two hours at reflux in a solvent selected from the aromatic hydrocarbons such as, in particular benzene, toluene and the mixtures thereof, at the rate of two moles of II for one mole of III, to obtain the corresponding 4-(alkylpiperidino)-butyronitrile of formula IV

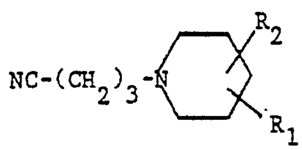

(IV)

(wherein $R_1$ and $R_2$ are as defined above) ; and

2°) said 4-(alkylpiperidino)-butyronitrile is reacted with the 1,3,5-trimethoxybenzene in the presence of a gaseous stream of HCl, at a temperature of between — 5 and + 5 °C, in an anhydrous solvent selected in particular from the group constituted by benzene, toluene, chlorobenzene and the mixtures thereof, at the rate of one mole of IV for one mole of 1,3,5-trimethoxybenzene, then the ketimine derivative thus obtained is subjected to a reaction of hydrolysis for at least 0.5 hour at the reflux temperature of the reaction medium.

**Patentansprüche** (für die Vertragsstaaten : BE, CH, DE, FR, GB, IT, LI, LU, NL, SE)

1. (2,4,6-Trimethoxyphenyl)-(3-piperidinopropyl)-keton-Derivat, dadurch gekennzeichnet, daß es ausgewählt ist aus der Gruppe bestehend aus

(i) den (2,4,6-Trimethoxyphenyl)-[3-(alkylpiperidino)-propyl]-ketonen der allgemeinen Formel I

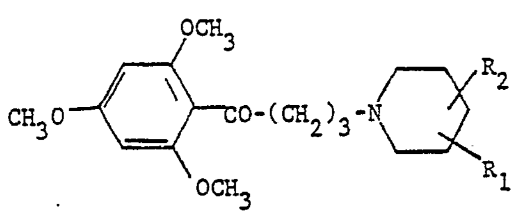

(I)

worin

$R_1$ eine Gruppe $CH_3$ oder $C_2H_5$ in der Position 2 oder 3 der Piperidinogruppe darstellt, und

$R_2$ für H, $CH_3$ oder $C_2H_5$ steht ; und (ii) deren Additionssalzen.

2. (2,4,6-Trimethoxyphenyl)-[3-(2-methylpiperidino)-propyl]-keton und seine Additionssalze.

3. (2,4,6-Trimethoxyphenyl)-[3-(3-methylpiperidino)-propyl]-keton und seine Additionssalze.

4. (2,4,6-Trimethoxyphenyl)-[3-(2-äthylpiperidino)-propyl]-keton und seine Additionssalze.

5. (2,4,6-Trimethoxyphenyl)-[3-(3,5-dimethylpiperidino)-propyl]-keton und seine Additionssalze.

6. (2,4,6-Trimethoxyphenyl)-[3-(2,6-dimethylpiperidino)-propyl]-keton und seine Additionssalze.

7. Therapeutische Zusammensetzung, dadurch gekennzeichnet, daß sie in Verbindung mit einem physiologisch akzeptablen Exzipienten mindestens ein (2,4,6-Trimethoxyphenyl)-[3-(alkylpiperidino)-propyl]-keton oder eines seiner nicht toxischen Additionssalze nach einem der Ansprüche 1 bis 6 enthält.

8. Verfahren zur Herstellung einer Verbindung der Formel I nach Anspruch 1, bei welchem man 4-(Alkylpiperidino)-butyronitril bildet, welches man mit 1,3,5-Trimethoxybenzol kondensiert, und das resultierende Ketiminderivat hydrolysiert, dadurch gekennzeichnet, daß man

1°) ein substituiertes Piperidin der Formel II

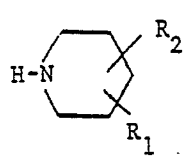

(II)

(worin $R_1$ und $R_2$ obige Bedeutung haben) mit dem 4-Chlorbutyronitril der Formel III

$$Cl-(CH_2)_3-CN \quad (III)$$

mindestens 2 h unter Rückfluß in einem Lösungsmittel, ausgewählt aus aromatischen Kohlenwasserstoffen, wie insbesondere Benzol, Toluol und Mischungen davon, in einer Menge von 2 Mol II pro 1 Mol III zur Gewinnung von 4-(Alkylpiperidino)-butyronitril der Formel IV

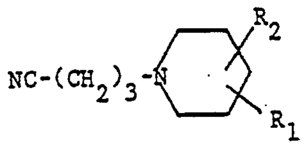

(IV)

(worin $R_1$ und $R_2$ obige Bedeutung haben) zur Umsetzung bringt ; und

2°) 4-(Alkylpiperidino)-butyronitril mit 1,3,5-Trimethoxybenzol in Gegenwart eines HCl-Gasstroms bei einer Temperatur zwischen —5 und +5 °C in einem wasserfreien Lösungsmittel, ausgewählt insbesondere aus der Gruppe bestehend aus Benzol, Toluol, Chlorbenzol und Mischungen davon, in einer Menge von 1 Mol IV pro 1 Mol 1,3,5-Trimethoxybenzol zur Umsetzung bringt und dann das so entstandene Ketiminderivat während mindestens 0,5 h bei Rückflußtemperatur des Reaktionsmilieus einer Hydrolysereaktion unterwirft.

**Patentansprüche** (für den Vertragsstaat AT)

1. Verwendung eines (2,4,6-Trimethoxyphenyl)-(3-piperidinopropyl)-keton-Derivats, ausgewählt aus der Gruppe bestehend aus

(i) den (2,4,6-Trimethoxyphenyl)-[3-(alkylpiperidino)-propyl]-ketonen der allgemeinen Formel I

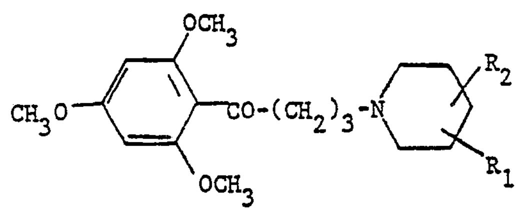

(I)

worin

$R_1$ eine Gruppe $CH_3$ oder $C_2H_5$ in der Position 2 oder 3 der Piperidinogruppe darstellt, und

$R_2$ für H, $CH_3$ oder $C_2H_5$ steht ; und (ii) deren nichttoxischen Additionssalzen

zur Herstellung eines Medikaments.

2. Verwendung nach Anspruch 1, dadurch gekennzeichnet, daß das Derivat (2,4,6-Trimethoxyphenyl)-[3-(2-methylpiperidino)-propyl]-keton und seine Additionssalze ist.

3. Verwendung nach Anspruch 1, dadurch gekennzeichnet, daß das Derivat (2,4,6-Trimethoxyphenyl)-[3-(3-methylpiperidino)-propyl]-keton und seine Additionssalze ist.

4. Verwendung nach Anspruch 1, dadurch gekennzeichnet, daß das Derivat (2,4,6-Trimethoxyphenyl)-[3-(2-Äthyl-piperidino)-propyl]-keton und seine Additionssalze ist.

5. Verwendung nach Anspruch 1, dadurch gekennzeichnet, daß das Derivat (2,4,6-Trimethoxyphenyl)-[3-(3,5-dimethylpiperidino)-propyl]-keton und seine Additionssalze ist.

6. Verwendung nach Anspruch 1, dadurch gekennzeichnet, daß das Derivat (2,4,6-Trimethoxyphenyl)-[3-(2,6-dimethylpiperidino)-propyl]-keton und seine Additionssalze ist.

7. Verwendung nach einem der Ansprüche 1 bis 6, dadurch gekennzeichnet, daß mindestens ein (2,4,6-Trimethoxyphenyl)-[3-(alkylpiperidino)-propyl]-keton-Derivat oder eines seiner nichttoxischen Additionssalze mit einem physiologisch akzeptablen Exzipienten kombiniert ist.

8. Verfahren zur Herstellung einer Verbindung der Formel I nach Anspruch 1, bei welchem man 4-(Alkylpiperidino)-butyronitril bildet, welches man mit 1,3,5-Trimethoxy-benzol kondensiert, und das resultierende Ketiminderivat hydrolysiert, dadurch gekennzeichnet, daß man

1°) ein substituiertes Piperidin der Formel II

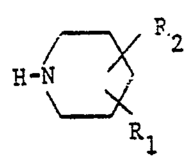

(II)

(worin $R_1$ und $R_2$ obige Bedeutung haben) mit dem 4-Chlorbutyronitril der Formel III

$$Cl—(CH_2)_3—CN \quad (III)$$

mindestens 2 h unter Rückfluß in einem Lösungsmittel, ausgewählt aus aromatischen Kohlenwasserstoffen, wie insbesondere Benzol, Toluol und Mischungen davon, in einer Menge von 2 Mol II pro 1 Mol III zur Gewinnung von 4-(Alkylpiperidino)-butyronitril der Formel IV

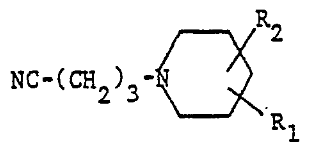

(IV)

(worin $R_1$ und $R_2$ obige Bedeutung haben) zur Umsetzung bringt ; und

2°) 4-(Alkylpiperidino)-butyronitril mit 1,3,5-Trimethoxybenzol in Gegenwart eines HCl-Gasstroms bei einer Temperatur zwischen —5 und +5 °C in einem wasserfreien Lösungsmittel, ausgewählt insbesondere aus der Gruppe bestehend aus Benzol, Toluol, Chlorbenzol und Mischungen davon, in einer Menge von 1 Mol IV pro 1 Mol 1,3,5-Trimethoxybenzol zur Umsetzung bringt und dann das so entstandene Ketiminderivat während mindestens 0,5 h bei Rückflußtemperatur des Reaktionsmilieus einer Hydrolysereaktion unterwirft.